# EUROPEAN PATENT APPLICATION

(11) **EP 2 851 033 A2**
(43) Date of publication of application: **25.03.2015**
(21) Application number: 14186185.6
(22) Date of filing: 24.09.2014
(51) Int. Cl.: A61B 19/08

(54) **An aseptic bag to encapsulate an energy source of a surgical instrument**

(30) Priority: 24.09.2013 US 201361881558 P; 21.08.2014 US 201414465050
(71) Applicant: Covidien LP, Mansfield, MA 02048 (US)
(72) Inventor: Craig, Jason L., Loveland, Colorado 80537 (US); Rousseau, Nicolas, 33410 Omet (FR)
(74) Representative: Hoffmann Eitle

(57) **Abstract**

A method of preparing a surgical system for surgery includes inserting a battery assembly into an inner chamber of an aseptic bag through an open end of the aseptic bag, positioning a portion of a housing of a surgical instrument through the open end of the aseptic bag, coupling the battery assembly to the surgical instrument, and sealing the open end of the aseptic bag. The battery assembly is coupled to the surgical instrument while the battery assembly is within the inner chamber of the aseptic bag. When the open end of the aseptic bag is sealed the battery assembly is encapsulated within the inner chamber of the aseptic bag. A generator of the surgical system may additionally or alternatively be encapsulated similarly.

## Description

### Background

### 1. Technical Field

The present disclosure relates to surgical instruments suitable for use in sterile environments and, more specifically, to surgical instruments having a non-sterile component encapsulated within a sterile membrane for use in a sterile environment.

### 2. Discussion of Related Art

After a surgical instrument has been manufactured and/or after a surgical instrument has been used during a surgical procedure, the surgical instrument can be subjected to physical sterilization and/or chemical sterilization in order to kill or eliminate transmissible agents. Physical sterilization can include gamma radiation sterilization which can be suitable in many circumstances. In some circumstances, however, gamma radiation can damage the components of the surgical instrument, in particular the electronic components thereof. As a result, the options available to sterilize such surgical instruments can be limited to heat or steam sterilization and/or chemical sterilization, such as ethylene oxide, ozone, and/or hydrogen peroxide, for example. While such options are suitable in many circumstances, these options may be more expensive, more time-consuming to perform, and/or unavailable at a particular facility. Further, components of the surgical instrument may degrade or be rendered inoperable after being subjected to one or more sterilizations.

### Summary

In accordance with the present disclosure, a surgical system is provided including a surgical instrument, a battery assembly, an aseptic bag, and a sealing member. The battery assembly is configured to deliver energy to the surgical instrument and is removably engagable with the surgical instrument. The aseptic bag includes an open end and an inner chamber. The battery assembly is positionable within the inner chamber of the aseptic bag. The sealing member is in contact with the aseptic bag and the housing of the surgical instrument to seal the open end of the aseptic bag to encapsulate the battery assembly within the inner chamber while still permitting energy delivery from the battery assembly to the surgical instrument. The surgical system may include a closure member affixed around the open end of the aseptic bag and affixed to the surgical instrument to seal the open end of the aseptic bag.

In aspects, the surgical system includes a generator configured to receive energy from the battery assembly and configured to deliver energy to the surgical instrument. The surgical instrument including a slot to slidably receive the generator. The generator being positionable within the inner chamber while slidably received within the slot. The sealing member may encapsulate the generator within the inner chamber of the aseptic bag.

In aspects, the aseptic bag includes a strap positioned adjacent to the first open end and the surgical instrument includes a handle assembly having a proximal portion. The strap is configured to engage the proximal portion of the handle assembly when the sealing member is in contact with the aseptic bag and the housing of the surgical instrument.

Another surgical system provided in accordance with the present disclosure includes a surgical instrument, an energy source, an aseptic bag, and a sealing member. The surgical instrument includes first electrical contacts. The energy source includes second electrical contacts configured to electrically couple to the first electrical contacts of the surgical instrument. The energy source delivers energy to the surgical instrument and is removably engagable with the surgical instrument. The surgical instrument can deliver the energy to tissue and/or use the energy to power the surgical instrument. The aseptic bag includes an open end and an inner chamber. The energy source is positionable within the inner chamber of the aseptic bag. The sealing member is affixed to the aseptic bag and configured to seal the open end of the aseptic bag to encapsulate the energy source within the inner chamber while still permitting energy delivery via the first and second electrical contacts.

The open end of the aseptic bag may be coupled to the surgical instrument such that the surgical instrument and the aseptic bag cooperate to encapsulate the energy source within the inner chamber. The aseptic bag can be coupled to the surgical instrument via adhesives and/or a snap-fit connector. The energy source delivers energy through the surface of the aseptic bag to the first electrical contacts of the surgical instrument. In aspects, the aseptic bag includes third electrical contacts that engage each of the first electrical contacts of the surgical instrument and the second electrical contacts of the energy source when the energy source is coupled to the surgical instrument and positioned within the inner chamber. In aspects, the energy source forms a handle of the surgical instrument when coupled to the surgical instrument. The surgical system can also include an adapter positioned between the energy source and the surgical instrument, the energy source coupled to the adapter and the adapter coupled to the surgical instrument.

In some aspects of the present disclosure, the sealing member has an adhesive surface. The adhesive may be on an inside surface of the aseptic bag. The sealing member may be a tab. When the sealing member is a tab, the tab can be affixed to the aseptic bag and include the adhesive surface. In particular aspects, the adhesive surface is protected by a releasable cover member. The releasable cover member can be removed to permit the adhesive surface to seal the open end of the aseptic bag.

Also provided in accordance with the present disclosure is a surgical system including a surgical instrument, an energy source, an aseptic bag, and a sealing member. The surgical instrument defines an attachment portion and includes first electrical contacts adjacent to the attachment portion. The surgical instrument can deliver the energy to tissue and/or use the energy to power the surgical instrument. The energy source is removably engages the attachment portion of the surgical instrument. The energy source includes second electrical contacts configured to electrically couple to the first electrical contacts of the energy source when the energy source engages the attachment portion of the surgical instrument. The energy source is configured to deliver energy to the surgical device through the first and second electrical contacts. The aseptic bag includes first and second open ends and an inner chamber between the first and second ends. The first open end is coupled to the surgical instrument. The first open end can be coupled to the surgical instrument near the electrical contacts of the surgical instrument such that the electrical contacts are within the inner chamber. The energy source is positionable within the inner chamber. The sealing member is affixed to the aseptic bag and configured to seal the second open end of the aseptic bag to encapsulate the energy source within the inner chamber.

In aspects, the surgical instrument includes an extended tab having an opening and the energy source includes a complimentary protrusion insertable within the opening to couple the energy source to the surgical instrument. In aspects, the energy source includes a release switch configured to engage the extended tab to release the protrusion from the opening. In certain aspects, the surgical system includes an energy source adapter positionable within the inner chamber. The energy source adapter can be between and coupled to each of the energy source and the surgical instrument. In particular aspects, the surgical instrument is a disposable surgical instrument.

Also provided in accordance with the present disclosure is a surgical kit for providing a sterile container for an energy source for a surgical instrument. The surgical kit includes an energy source and an aseptic bag sealed within a membrane. The energy source is configured to deliver energy through electrical contacts and configured to removably couple to a surgical instrument. The aseptic bag includes an open end, a sealing member, and electrical contacts. The inner chamber sized to receive the energy source. The adhesive surface including a releasable cover member and configured to seal the open end of the aseptic bag to seal the energy source within the inner chamber of the aseptic bag.

The aseptic bag may be folded or rolled within the kit. The aseptic bag may also be configured to couple to a portion of the surgical instrument. In aspects, the aseptic bag includes electrical contacts configure to engage electrical contacts of the surgical instrument and engage the energy source to deliver energy from the energy source to the surgical instrument. In some aspects, the aseptic bag includes a second open end configured to couple to the portion of the surgical instrument. The second open end may include a second adhesive surface.

Methods of preparing a surgical system for surgery are also provided in accordance with the present disclosure. Such methods include inserting a battery assembly into the inner chamber of the aseptic bag through an open end of the aseptic bag, positioning a portion of a housing of a surgical instrument through the open end of the aseptic bag, coupling the battery assembly to the surgical instrument while the battery assembly is within the inner chamber, and sealing the open end of the aseptic bag to the housing of the surgical instrument. Sealing the open end of the aseptic bag encapsulates the battery assembly within the inner chamber of the aseptic bag. The method may include affixing a closure member to around the open end of the aseptic bag to seal the open end of the aseptic bag to the surgical instrument. The method may include removing the surgical instrument and the aseptic bag from a surgical kit before inserting a battery assembly into the inner chamber of the aseptic bag.

The method may include inserting a generator into an inner chamber of the aseptic bag through the open end of the aseptic bag before sealing the open end of the aseptic bag and sealing the open end of the aseptic bag further includes encapsulating the generator within the inner chamber of the aseptic bag. The method may also include securing the generator in a slot defined by the housing of the surgical instrument after positioning a portion of a housing of a surgical instrument through the open end of the aseptic bag.

The method may include wrapping the battery assembly and a portion of the aseptic bag with a handle wrapper after sealing the open end of the aseptic bag to the housing of the surgical instrument. The handle wrapper may include an adhesive surface and wrapping the battery assembly may include affixing the adhesive surface of the handle wrapper to another portion of the handle wrapper to secure the handle wrapper around the battery assembly and a portion of the aseptic bag. The method may also include gathering loose portions of the aseptic bag around the battery assembly after coupling the battery assembly to the surgical instrument and before wrapping the battery assembly and a portion of the aseptic bag with the handle wrapper.

In aspects of the present disclosure, the open end of the aseptic bag includes a strap and positioning the portion of the housing of the surgical instrument through the open end of the aseptic bag includes positioning the strap over a proximal portion of a handle assembly of the surgical instrument to bias the open end proximally.

In still yet another aspect of the present disclosure, a method for providing a sterile surgical instrument is disclosed. The method includes providing a surgical instrument, coupling an energy source to the surgical instrument, extending an open end of an aseptic bag over the energy source, and sealing the open end.

The surgical instrument provided includes electrical contacts. The surgical instrument may be a disposable surgical instrument. In aspects, the method includes energizing the surgical instrument to deliver energy from the energy source to tissue with an end effector of the surgical instrument.

Coupling the energy source includes engaging the electrical contacts of the surgical instrument with the electrical contacts of the energy source. The aseptic bag may be attached to the surgical instrument such that the energy source couples to the surgical instrument with the aseptic bag therebetween. The aseptic bag may also include electrical contacts such that the electrical contacts of the aseptic bag engage the electrical contacts of the surgical instrument.

In some aspects of the present disclosure, the adhesive surface is a tab including a releasable cover member. The aseptic bag may be extended by pulling on the tab. Sealing may include removing the releasable cover member from the tab.

Further, to the extent consistent, any of the aspects described herein may be used in conjunction with any or all of the other aspects described herein.

### Brief Description of the Drawings

Various aspects of the present disclosure are described hereinbelow with reference to the drawings, wherein:
FIG. 1 is a side, perspective view of a portable, battery-powered surgical system configured for use in accordance with the present disclosure;
FIG. 2 is a side, perspective view of another portable, battery-powered surgical system configured for use in accordance with the present disclosure;
FIG. 3 is a side, perspective view of a battery assembly provided in accordance with the present disclosure and configured for use with either or both of the instruments of the systems of FIGS. 1 and 2;
FIG. 4 is an exploded, perspective view of the battery assembly of FIG. 3;
FIG. 5 is perspective view of an embodiment of a surgical system provided in accordance with the present disclosure, shown with the battery assembly disengaged from the handle of the surgical instrument;
FIG. 6 is a bottom perspective view of the handle of the surgical instrument of FIG. 5;
FIG. 7 is a perspective view of the surgical system of FIG. 5 with the battery assembly coupled to the handle of the surgical instrument;
FIG. 8 is a perspective view of the handle portion of the surgical system of FIG. 5 with the aseptic bag extended over the battery assembly;
FIG. 9 is a perspective view of the handle portion of the surgical system of FIG. 5 with the open end of the aseptic bag sealed about the battery assembly;
FIG. 10 is perspective view of another embodiment of a surgical system provided in accordance with the present disclosure, wherein the battery assembly and aseptic bag are shown disengaged from the handle of the surgical instrument and one another;
FIG. 11 is a perspective view of the surgical system of FIG. 10 with the battery assembly coupled to handle of the surgical instrument and sealed within the inner chamber of the aseptic bag;
FIG. 12 is perspective view of another embodiment of a surgical system provided in accordance with the present disclosure, wherein the battery assembly is disengaged from the handle of the surgical instrument;
FIG. 13 is perspective view of another embodiment of a surgical system provided in accordance with the present disclosure, wherein the battery assembly is disengaged from the handle of the surgical instrument;
FIG. 14 is a perspective view of the surgical system of FIG. 13, with the battery assembly coupled to the handle of the surgical instrument and the open end of the aseptic bag extended over the battery assembly;
FIG. 15 is a perspective view of the surgical system of FIG. 14 with the open end of the aseptic bag sealed about the battery assembly;
FIG. 16 is a perspective view of another embodiment of a surgical system provided in accordance with the present disclosure, wherein the aseptic bag is affixed to the adapter and the adapter and the battery assembly are disengaged from one another and the handle of the surgical instrument;
FIG. 17 is a perspective view of the surgical system of FIG. 16 with the adapter engaged with the handle of the surgical instrument;
FIG. 18 is a perspective view of the surgical system of FIG. 16 with the adapter engaged with the handle of the surgical instrument and the battery assembly engaged to the adapter with the aseptic bag encapsulating the battery assembly;
FIG. 19 is a perspective view of a kit provided in accordance with the present disclosure including sterile components of a surgical system sealed within a membrane;
FIG. 20 is a perspective view of the aseptic bag of the kit of FIG. 19;
FIG. 21 is a perspective view of a battery assembly being inserted through the opening of the aseptic bag of FIG. 19;
FIG. 22 is a side view of the aseptic bag of FIG. 19 with a battery assembly and a generator within the inner chamber thereof;
FIG. 23 is a side view of the instrument of the kit of FIG. 19 with a proximal portion of the housing being inserted through the opening of the aseptic bag of FIG. 19;
FIG. 24 is a side view of the instrument of FIG. 19 with the proximal portion of the housing within the inner chamber of the aseptic bag of FIG. 19;
FIG. 25 is a side view of a generator being attached to the instrument of FIG. 19;
FIG. 26 is a side view of a battery assembly being attached to the instrument of FIG. 19;
FIG. 27 is a side view of the surgical instrument of FIG. 19 with the generator and the battery assembly attached with the handle wrap of the kit of FIG. 19 wrapped around the battery assembly;
FIG. 28 is a side view of the assembled surgical system of FIG. 19 with the opening of the aseptic bag sealed with the closure member of the kit of FIG. 19;
FIG. 29 is a perspective view of another kit provided in accordance with the present disclosure; and
FIG. 30 is a perspective view of yet another kit provided in accordance with the present disclosure.

### Detailed Description

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" will refer to the portion of the device or component thereof that is closest to the clinician and the term "distal" will refer to the portion of the device or component thereof that is furthest from the clinician.

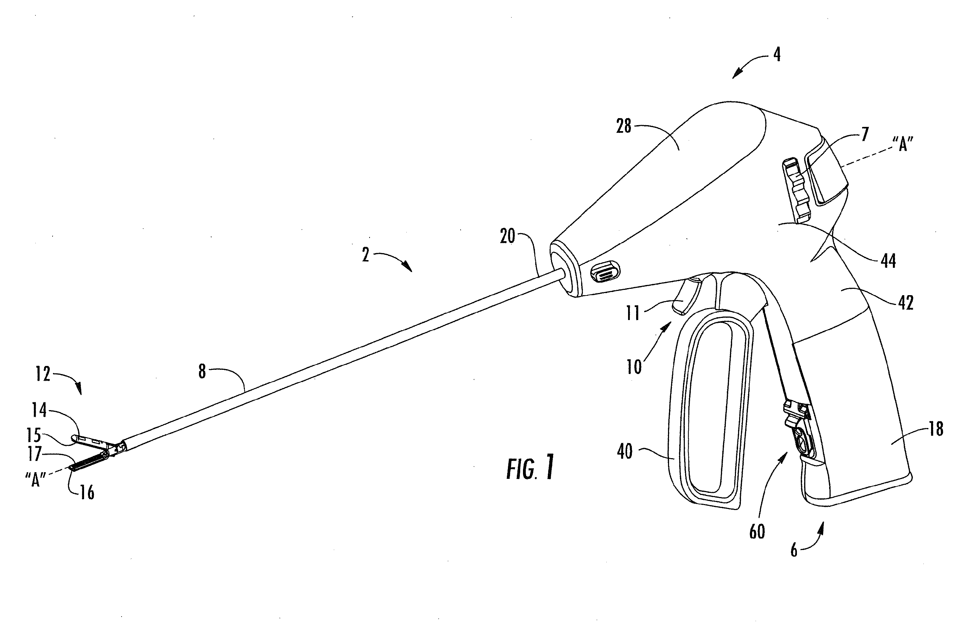

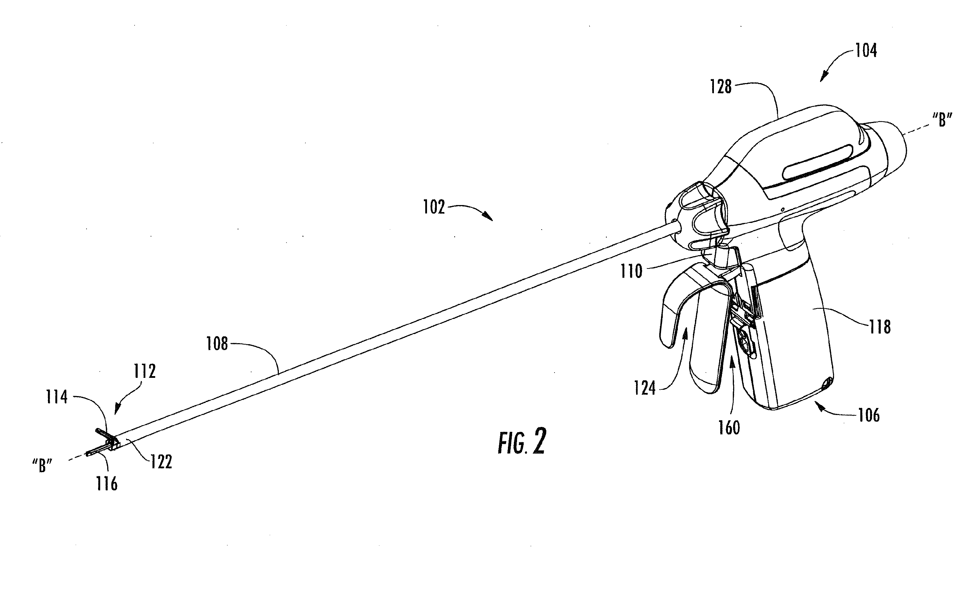

Referring now to FIGS. 1 and 2, FIG. 1 depicts a portable, battery-powered electrosurgical instrument 2 and FIG. 2 depicts a portable, battery-powered ultrasonic surgical instrument 102. For the purposes herein, any electrosurgical instrument, e.g., instrument 2; ultrasonic instrument, e.g., instrument 102; or any other suitable battery-powered device, e.g., a surgical instrument, handheld tool, electronic device, or the like, may be utilized in accordance with the present disclosure. Obviously, different considerations apply to each particular type of device; however, the features and aspects of the present disclosure are equally applicable and remain generally consistent with respect to any suitable battery-powered device. For the purposes herein, electrosurgical instrument 2 and ultrasonic instrument 102 are generally described.

With reference to FIG. 1, electrosurgical instrument 2, shown as an electrosurgical forceps, generally includes a housing 4, a battery assembly 18, an electrosurgical generator 28, a handle assembly 6, a rotating assembly 7, a shaft 8, a trigger assembly 10, a drive assembly (not shown), and an end effector assembly 12. End effector assembly 12 operatively connects to handle assembly 6 via the drive assembly (not shown) for imparting movement of one or both of jaw members 14, 16 of end effector assembly 12 between a spaced-apart position and an approximated position for grasping tissue therebetween.

Continuing with reference to FIG. 1, shaft 8 is coupled to housing 4 at proximal end 20 thereof and extends distally from housing 4 to define a longitudinal axis "A-A." End effector assembly 12, including jaw members 14 and 16, is disposed at a distal end 22 of shaft 8. End effector assembly 12 is shown configured as a unilateral assembly wherein jaw member 16 is fixed relative to shaft 8 and jaw member 14 is pivotable relative to jaw member 16 and shaft 8 between the spaced-apart and approximated positions. However, this configuration may be reversed, e.g., wherein jaw member 14 is fixed relative to shaft 8 and jaw member 16 is pivotable relative to jaw member 14 and shaft 8. Alternatively, end effector assembly 12 may be configured as a bilateral assembly, e.g., wherein both jaw members 14, 16 are pivotable relative to one another and shaft 8 between the spaced-apart and approximated positions.

Electrosurgical instrument 2 may be configured as a bipolar instrument. That is, each of the jaw members 14, 16 may include a respective seal plate 15, 17 that is configured to function as an active (or activatable) and/or return electrode. Each seal plate 15, 17 is electrically coupled to generator 28 via one or more electrical leads (not shown) that extend from generator 28, through shaft 8, and eventually coupling to one or both of seal plates 15, 17 for conducting energy through tissue grasped therebetween. However, forceps 2 may alternatively be configured as a monopolar instrument.

Handle assembly 6 includes a moveable handle 40 that is movable relative to fixed handle portion 42 for moving jaw members 14, 16 of end effector assembly 12 between the spaced-apart and approximated positions. Rotating assembly 7 is rotatable in either direction about longitudinal axis "A-A" to rotate shaft 8 and, thus, end effector assembly 12 about longitudinal axis "A-A." Trigger assembly 10 is in operable communication with a knife assembly (not shown) including a knife blade (not shown) that is selectively translatable between jaw members 14, 16 to cut tissue grasped therebetween, e.g., upon actuation of trigger 11 of trigger assembly 10.

With continued reference to FIG. 1, housing 4 is configured to releasably engage both of the energy sources of instrument 2, e.g., electrosurgical generator 28 and battery assembly 18. Generator 28 is releasably engagable with body portion 44 of housing 4, while battery assembly 18 is releasably engagable with fixed handle portion 42 of housing 4. More specifically, battery assembly 18 is configured to engage fixed handle portion 42 of housing 4 such that battery assembly 18 functions as the stationary handle of housing 4 to facilitate grasping of the forceps 2. Generator 28 releasably engages body portion 44 of housing 4 and may be selectively removable from body portion 44 either in connection with the removal of battery assembly 18 or independently.

When forceps 2 is assembled, generator 28 is disposed in operable electrical communication with battery assembly 18 to provide electrosurgical energy to end effector 12 for electrosurgically treating tissue, e.g., to seal tissue, although forceps 2 may alternatively be configured to deliver any other suitable form of energy to tissue, e.g., thermal energy, microwave energy, light energy, etc. With respect to electrosurgical tissue treatment, generator 28 may include suitable electronics that convert the electrical energy from battery assembly 18 into an RF energy waveform to energize one or both of jaw members 14, 16. That is, generator 28 may be configured to transmit RF energy to seal plate 15 of jaw member 14 and/or seal plate 17 of jaw member 16 to conduct energy therebetween for treating tissue. An activation switch is disposed on housing 4 activatable for selectively enabling generator 28 to generate and subsequently transmit RF energy to seal plate 15 and/or seal plate 17 of jaw members 14, 16, respectively, for treating tissue grasped therebetween.

Referring now to FIG. 2, ultrasonic instrument 102 includes components similar to that of electrosurgical instrument 2 shown in Fig. 1, namely, a housing 104, a battery assembly 118, an electrosurgical generator 128, a handle assembly 106, a shaft 108, and an end effector assembly 112. Accordingly, only the differences between ultrasonic instrument 102 and electrosurgical instrument 2 (FIG. 1) will be described in detail below.

Housing 104 is configured to releasably engage ultrasonic generator 128 and battery assembly 118. Shaft 108 extends distally from housing 104 to define longitudinal axis "B-B" and includes end effector assembly 112 disposed at distal end 122 thereof. One or both of jaw members 114 and 116 of end effector assembly 112 are movable relative to one another, e.g., upon actuation of moveable handle 124, between an open position and a clamping position for grasping tissue therebetween. Further, one of the jaw members, e.g., jaw member 116, serves as an active or oscillating ultrasonic blade that is selectively activatable to ultrasonically treat tissue grasped between jaw members 114, 116.

Generator 128 includes a transducer (not shown) configured to convert electrical energy provided by battery assembly 118 into mechanical energy that produces motion at the end of a waveguide, e.g., at blade 116. More specifically, the electronics (not explicitly shown) of the generator 128 convert the electrical energy provided by battery assembly 118 into a high voltage AC waveform that drives the transducer (not shown). When the transducer (not shown) and the waveguide are driven at their resonant frequency, mechanical, e.g., ultrasonic, motion is produced at the active jaw member 116 for treating tissue grasped between jaw members 114, 116. Further, an activation button 110 disposed on housing 104 is selectively activatable to operate instrument 102 in two modes of operation: a low-power mode of operation and a high-power mode of operation.

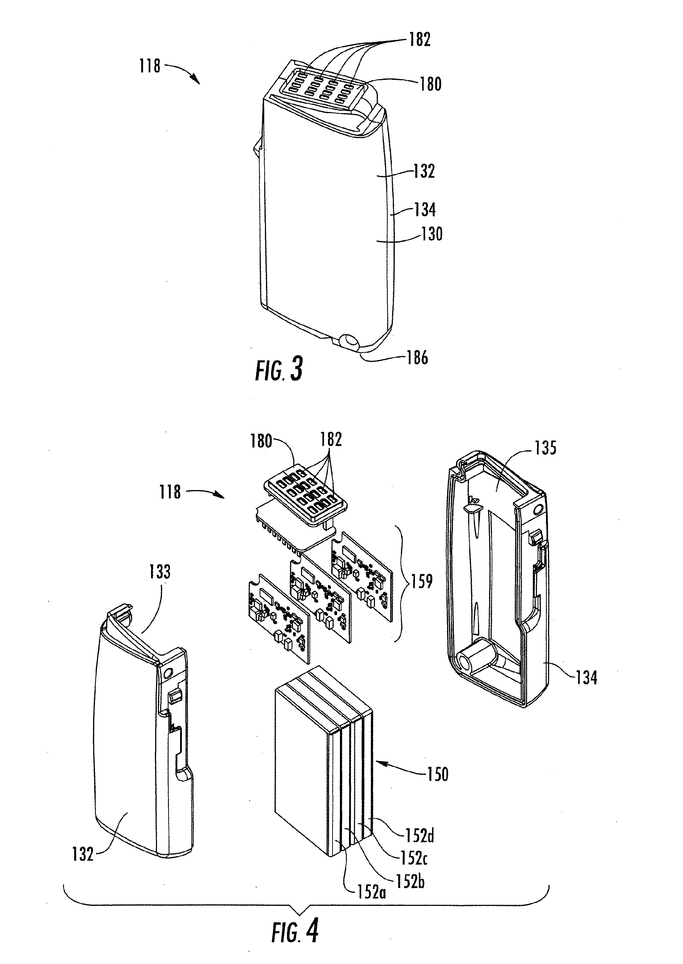

With reference to FIGS. 3 and 4, battery assembly 118 generally includes an outer housing 130, a battery pack 150, battery circuitry 159, and a contact cap 180. Battery assembly 18 of electrosurgical instrument 2 (FIG. 1) may be configured similarly to battery assembly 118 and, thus, will not be described herein for purposes of brevity.

Outer housing 130 of battery assembly 118 is formed from first and second housing parts 132, 134 that cooperate to house battery pack 150 and battery circuitry 159. Housing parts 132, 134 define cut-outs 133, 135, respectively, that cooperate to form a window configured to retain contact cap 180. Contact cap 180 is electrically coupled to battery circuitry 159, which, in turn, is electrically coupled to battery pack 140. Contact cap 180 includes electrical contacts 182 configured to provide an electrical interface between battery assembly 118, e.g., battery pack 150 and battery circuitry 159, and both the battery-powered device, e.g., electrosurgical instrument 2 (FIG. 1) or ultrasonic instrument 102 (FIG. 2), and a battery charging device (not shown) for transmitting energy and/or control signals therebetween. Battery pack 150 includes a plurality of battery cell assemblies 152a, 152b, 152c, 152d, e.g., four (4) battery cell assemblies 152a-152d, although greater or fewer battery cell assemblies 152a-152d are also contemplated.

As used herein the battery assembly, electrosurgical generator, or any other energy source or removable electrical component of instruments 2, 102 may be provided for use as described below but for exemplary purposes only the battery assemblies are described in detail below.

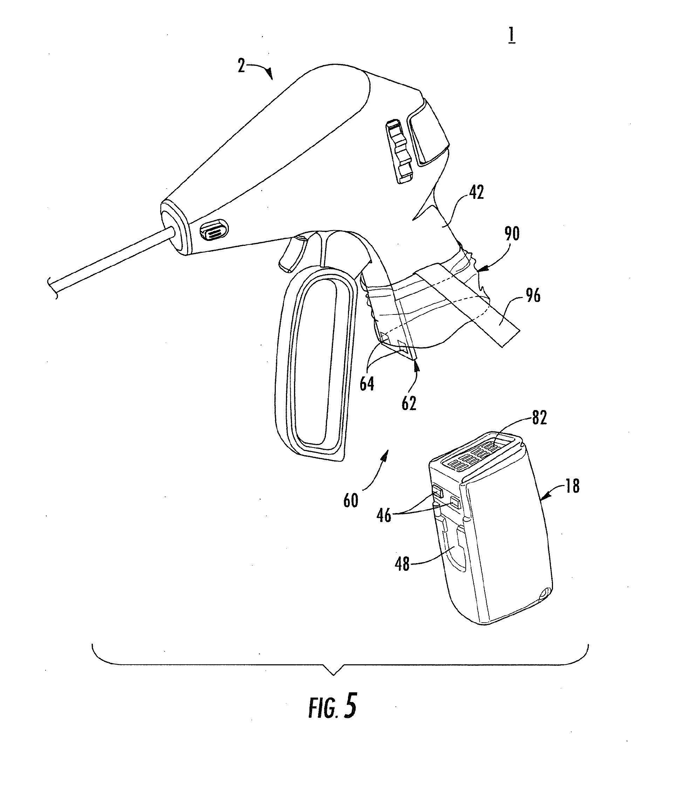

Referring to FIGS. 5-9, one embodiment of a surgical system 1 provided in accordance with the present disclosure incorporates surgical instrument 2 (or any other suitable surgical instrument), battery assembly 18 (or any other suitable energy source), an aseptic bag 90, and a sealing member 96. Surgical instrument 2 includes a fixed handle 42 including first electrical contacts 45. Battery assembly 18 is configured to deliver energy to instrument 2 from second electrical contacts 82 to first electrical contacts 45. The energy received by instrument 2 may be used to power instrument 2 and/or be delivered to tissue by surgical instrument 2 to treat tissue, as detailed above.

Battery assembly 18 is configured to releasably couple to fixed handle 42 and includes second electrical contacts 82. In embodiments, surgical system I includes a coupling assembly 60 configured to secure battery assembly 18 to fixed handle 42. Coupling assembly 60 includes an extended tab 62 extending from fixed handle 42. Extended tab 62 can include an opening or notch 64 configured to receive a detent 46 of battery assembly 18 to snap-fit battery assembly 18 and fixed handle 42 to one another. Battery assembly 18 can include a release mechanism 48 to disengage detent 46 from within opening 64 thereby releasing battery assembly 18 from fixed handle 42. Other suitable releasable engagement mechanisms are also contemplated, e.g., friction-fitting, latching, pin-aperture coupling, magnetic, etc.

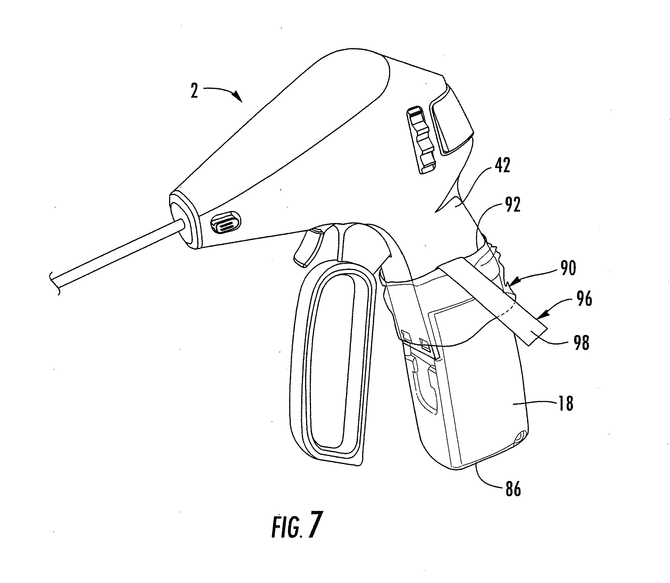

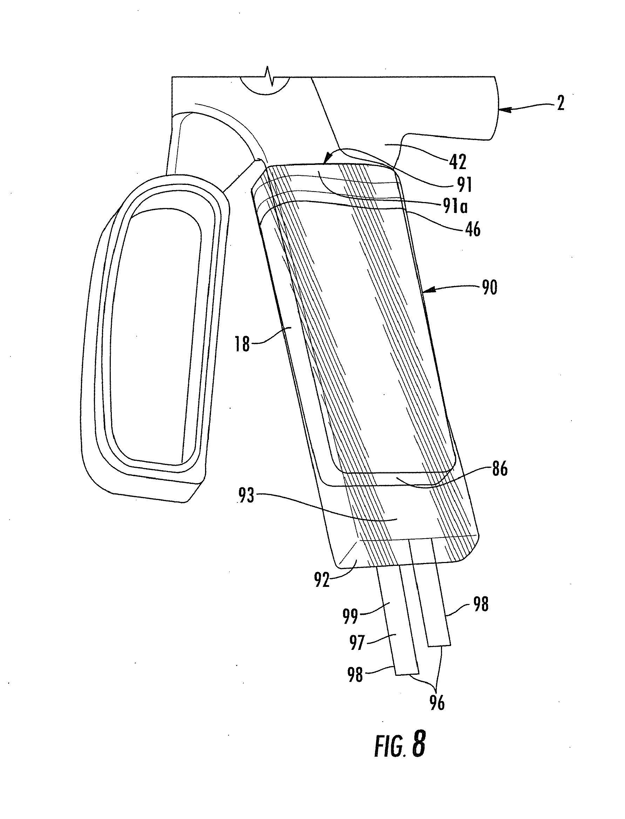

As best shown in FIG. 8, aseptic bag 90 defines a generally tubular configuration (although other configurations are also contemplated) and includes a first open end 91, a second open end 92, and an inner chamber 93. First open end 91 is affixed in a sealing relationship to fixed handle 42 of instrument 2. First open end 91 can be affixed to fixed handle 42 via an adhesive, a snap-fit connection, or any other suitable sealed coupling or combination thereof. More specifically, in some embodiments, an adhesive 91a is disposed on an inner surface of first open end 91 to adhere first open end 91 to fixed handle 42. Alternatively, or additionally, a band (not shown) configured to snap-fit on fixed handle 42 with first open end 91 between the band and fixed handle 42 to affix first open end 91 to fixed handle 42. In certain embodiments, the band seats in a groove (not shown) on fixed handle 42. As shown in FIG. 7, aseptic bag 90 can be rolled up or folded on fixed handle 42 such that second open end 92 is disposed over first open end 91. This low-profile configuration keeps aseptic bag 90 from interfering with the engagement of battery assembly 18 with fixed handle 42.

Referring again to FIGS. 5-9, sealing member 96 is affixed to a portion of aseptic bag 90 near second open end 92. Sealing member 96 is configured to seal second open end 92 when battery assembly 18 is positioned within inner chamber 93 defined between first and second open ends 91, 92. In embodiments, sealing member 96 includes an adhesive surface 97 (FIG. 8). Adhesive surface 97 can be disposed on an inner and/or outer surface of aseptic bag 90. In some embodiments, adhesive surface 97 includes a releasable cover member 99 configured to protect adhesive surface 97 from inadvertently adhering to another surface prematurely. It will be appreciated that releasable cover member 99 is removed prior to adhering adhesive surface 97 to fixed handle 42 to expose adhesive surface 97. In certain embodiments, sealing member 96 is a tab 98 affixed to aseptic bag 90 near second open end 92. Sealing member 96 can include more than one tab 98. In particular embodiments, tab 98 includes adhesive surface 97 and releasable cover member 99. Tab 98 can be used by a clinician to extend aseptic bag 90 as detailed below.

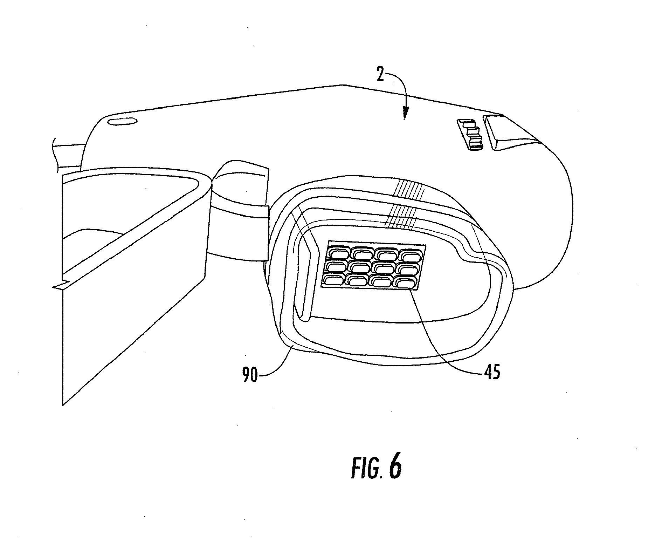

Referring again to FIG. 7, initially first open end 91 (FIG. 8) of aseptic bag 90 is affixed in a sealing relationship on fixed handle 42 of instrument 2. Second open end 92 is rolled up or folded upon fixed handle 42 and disposed over first open end 91 in a low-profile configuration. Battery assembly 18 is coupled to fixed handle 42 such that second electrical contacts 82 (FIG. 5) of battery assembly 18 electrically engage first electrical contacts 45 (FIG. 6) of instrument 2 and is mechanically engaged to fixed handle 42 via the engagement of notches and detents 64, 46, respectively.

Referring to FIG. 8, aseptic bag 90 is extended over battery assembly 18 fully disposing battery assembly 18 within inner chamber 93 of aseptic bag 90. In embodiments, tab 98 is pulled by a clinician to extend aseptic bag 90. Aseptic bag 90 unrolls or unfolds as it extends over battery assembly 18.

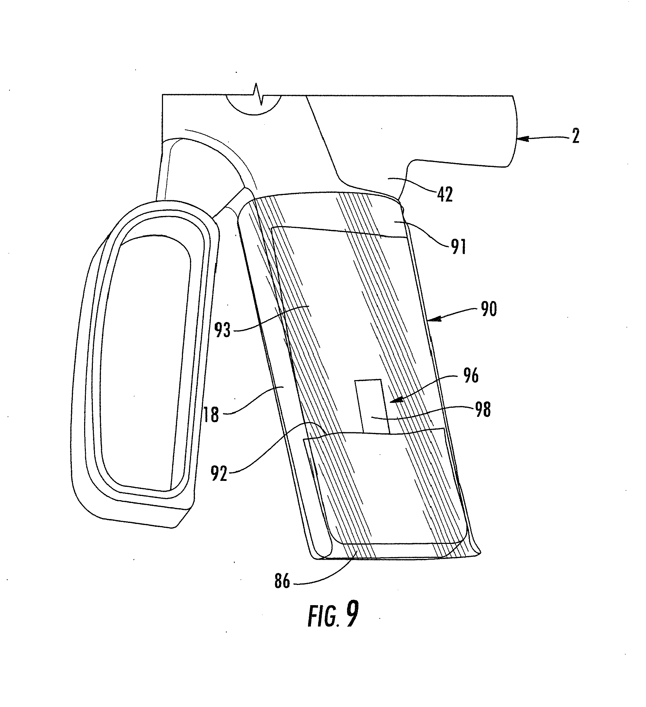

As shown in FIG. 9, when battery assembly 18 is disposed within inner chamber 93, second open end 92 is sealed with sealing member 96 sealing or encapsulating battery assembly 18 within inner chamber 93. In embodiments, adhesive surface 97 seals second open end 92. In some embodiments, releasable cover member 99 is removed from adhesive surface 97 to seal second open end 92. In certain embodiments, when aseptic bag 90 includes tab 98, adhesive surface 97 is disposed on tab 98. In particular embodiments, releasable cover member 99 provides visual indicia of the location of adhesive surface 97. Second open end 92 of aseptic bag 90 may fold over an end 86 of battery assembly 18 opposite second electrical contacts 82 (FIG. 3) and extends back towards fixed handle 42 to reduce excess material extending from battery assembly 18.

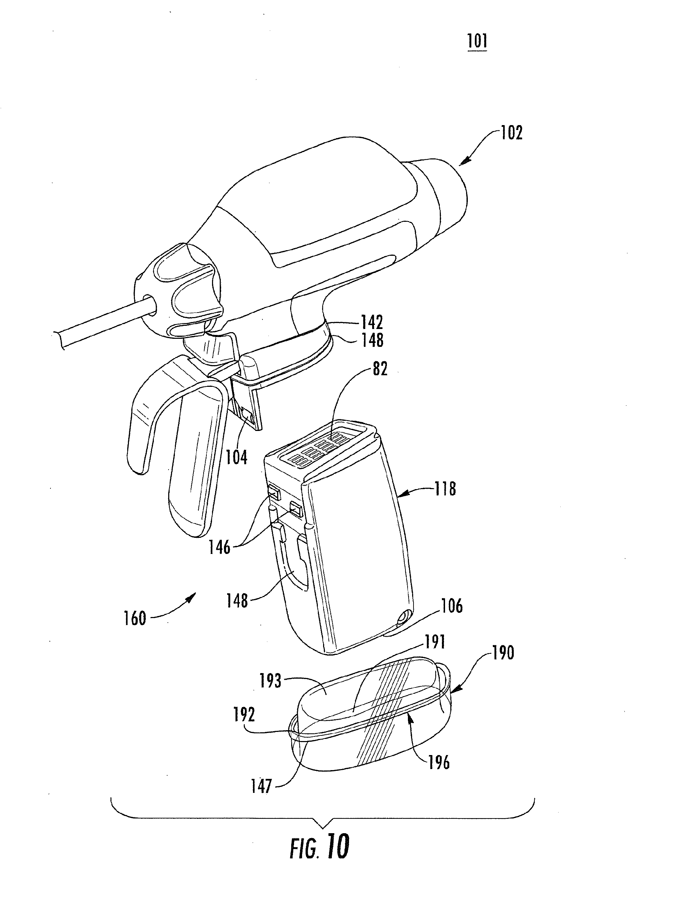

Referring to FIGS. 10-11, another embodiment of a surgical system 101 provided in accordance with the present disclosure incorporating surgical instrument 102 (or any other suitable surgical instrument), battery assembly 118 (or any other suitable energy source), an aseptic bag 190, and a sealing member 196. Instrument 102, battery assembly 118, aseptic bag 190, and sealing member 196 of surgical system 101 are substantially similar to instrument 2, battery assembly 18, aseptic bag 90, and sealing member 96 of surgical system 1 described above, and, as such, detailed discussion of surgical system 101 below will be limited to only the differences.

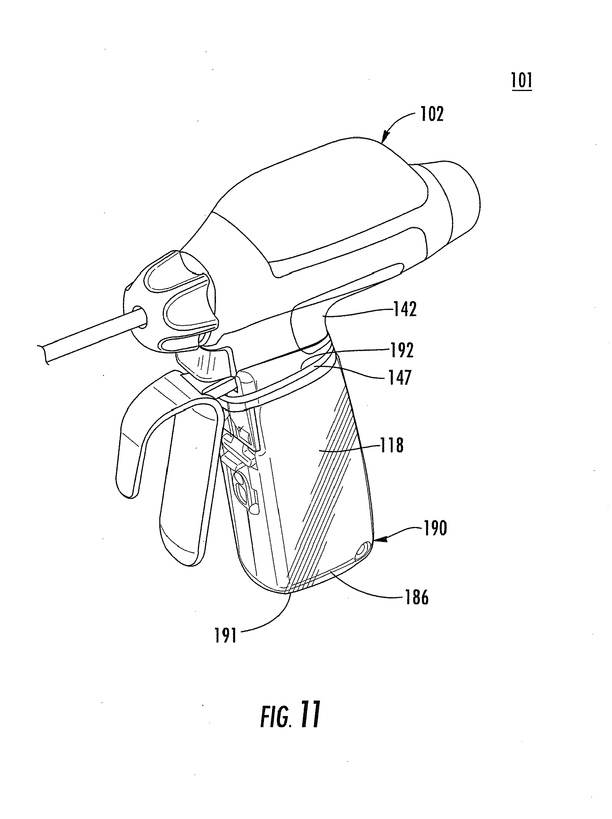

Aseptic bag 190 includes a closed end 191, an open end 192, and an inner chamber 193. Closed end 191 is sized and configured to receive an end 186 (FIG. 11) of battery assembly 118 opposite second electrical contacts 82. Open end 192 is configured to seal around fixed handle 142. Inner chamber 193 is sized and configured to receive battery assembly 118 therein.

Sealing member 196 is configured to seal open end 192 when battery assembly 118 is positioned within inner chamber 193. Sealing member 196 is affixed to aseptic bag 190 near open end 192 and configured to affix to fixed handle 142 in a sealing relationship. In embodiments, sealing member 196 is integral to open end 192. In some embodiments, sealing member 196 includes a band 147 configured to snap-fit on fixed handle 142 with open end 192 sealed by band 147. In certain embodiments, fixed handle 142 includes a groove 148 configured to receive band 147. For example, a clinician may use aseptic bag 190 by placing end 186 of battery assembly 118 in closed end 91 then unrolling aseptic bag 190 over battery assembly 118 until band 147 seats in groove 148, thus, encapsulating battery assembly 118 within inner chamber 193 of aseptic bag 190. It will be appreciated that sealing member 196 can include both adhesive surface 197 and band 147.

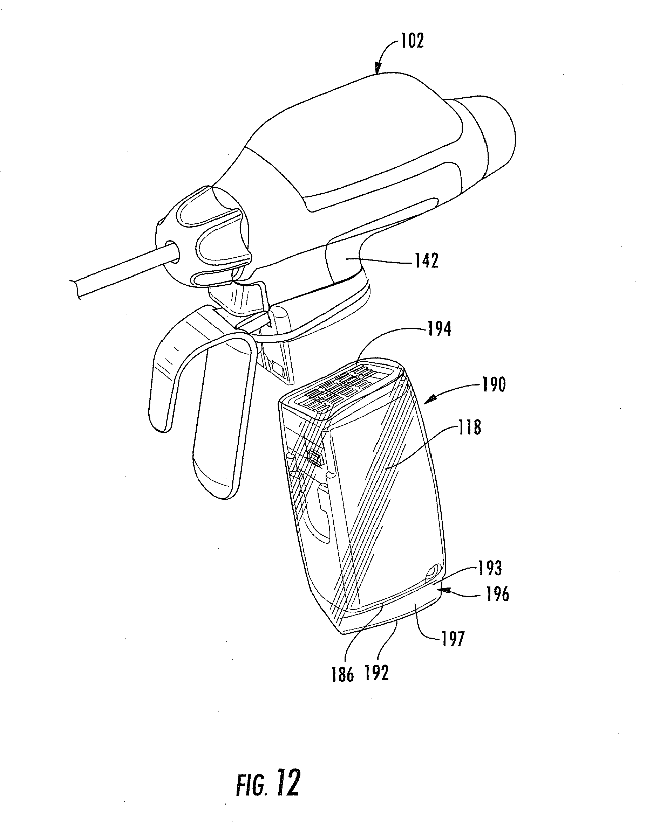

Referring to FIG. 12, in another embodiment of surgical system provided in accordance with the present disclosure includes battery assembly 118 encapsulated within inner chamber 193 of aseptic bag 190 before battery assembly 118 is coupled to surgical instrument 102. More specifically, closed end 191 of aseptic bag 190 receives battery assembly 118 such that second electrical contacts 182 (FIG. 3) engage third electrical contacts 194 extending through aseptic bag 190. Third electrical contacts 194 are configured to electrically couple first electrical contacts of handle 142 (similar to contacts 45 (FIG. 6)) with second electrical contacts 182 (FIG. 3) of battery assembly 118 through aseptic bag 190. With second electrical contacts 182 (FIG. 3) received within closed end 191, open end 192 is extended over battery assembly 118 fully disposing battery assembly 118 within inner chamber 193 of aseptic bag 191.

As shown in FIG. 12, sealing member 196 of aseptic bag 190 seals open end 192. Sealing member 196 includes an adhesive surface 197 and may further include a releasable cover member, similarly as detailed above. Once exposed, e.g., via removing the releasable cover member, adhesive surface 197 is brought into contact with a portion of aseptic bag 190 such that open end 192 is sealed to encapsulate battery assembly 118 within inner chamber 193 of aseptic bag 190. In some embodiments, open end 192 is folded over an end 186 of battery assembly 118 towards second electrical contacts 182 (FIG. 3) to encapsulate battery assembly 118 within inner chamber 193 of aseptic bag 190, as discussed above. After battery assembly 118 is encapsulated within aseptic bag 190, battery assembly 118 is coupled to fixed handle 142 of instrument 102. It will be appreciated that by sealing battery assembly 118 in aseptic bag 190 before coupling battery assembly 118 to instrument 102, a clinician is able to replace the battery assembly with a different battery assembly while in the operating theater.

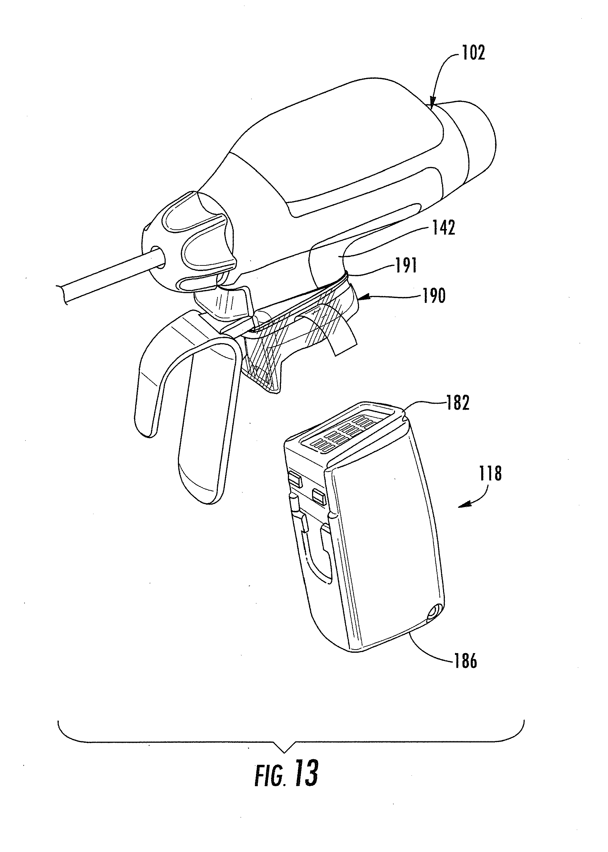

In another embodiment of a surgical system provided in accordance with the present disclosure and illustrated in FIGS. 13-15, closed end 191 of aseptic bag 190 is affixed to fixed handle 142. Closed end 191 can be affixed to fixed handle 142 using an adhesive connection and/or a snap-fit connection, similar to first open end 91 of aseptic bag 90 discussed above and illustrated in FIG. 7. In embodiments, aseptic bag 190 includes third electrical contacts 194 (FIG. 12) extending therethrough that are configured to electrically couple the first electrical contacts of handle 142 and electrical contacts 182 of battery assembly 118 with one another through aseptic bag 190 when battery assembly 118 is coupled to instrument 102.

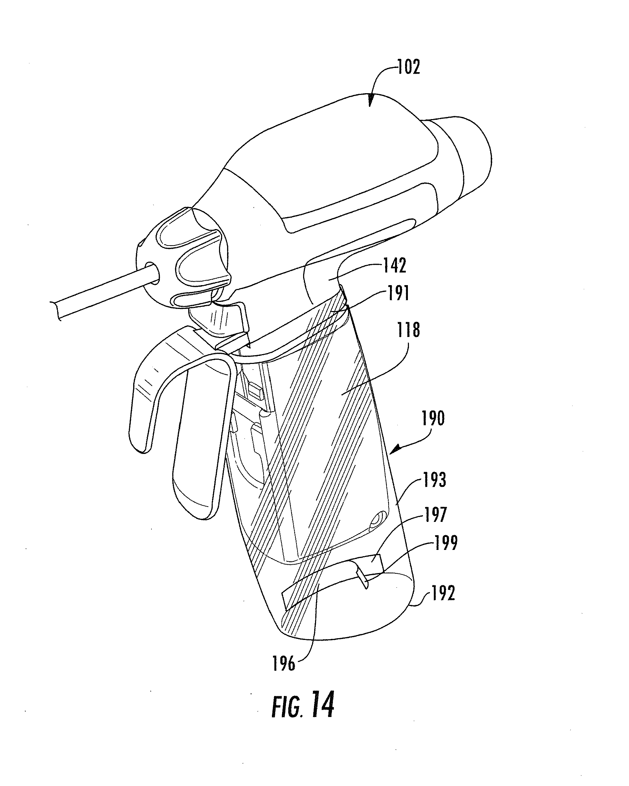

Referring to FIG. 14, battery assembly 118 is coupled to fixed handle 142 such that second electrical contacts 182 (FIG. 13) of battery assembly 118 electrically engage third electrical contacts 194 (FIG. 12) of aseptic bag 190. Open end 192 of aseptic bag 190 is then (or prior thereto) extended over battery assembly 118 fully disposing battery assembly 118 within inner chamber 193.

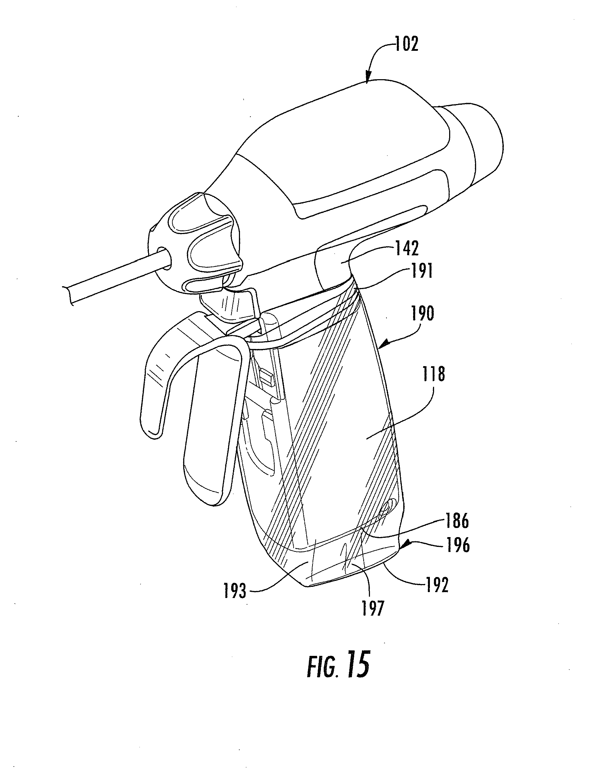

When battery assembly 118 is within inner chamber 193, sealing member 196 is sealed to seal open end 192 and encapsulate battery assembly 118 within aseptic bag 190 as shown in FIG. 15 and similarly as detailed above. In embodiments, releasable cover member 199 is removed from adhesive surface 197 to expose adhesive surface 197 to seal open end 192. In some embodiments, open end 192 is folded over end 186 of battery assembly 118 and towards fixed handle 142.

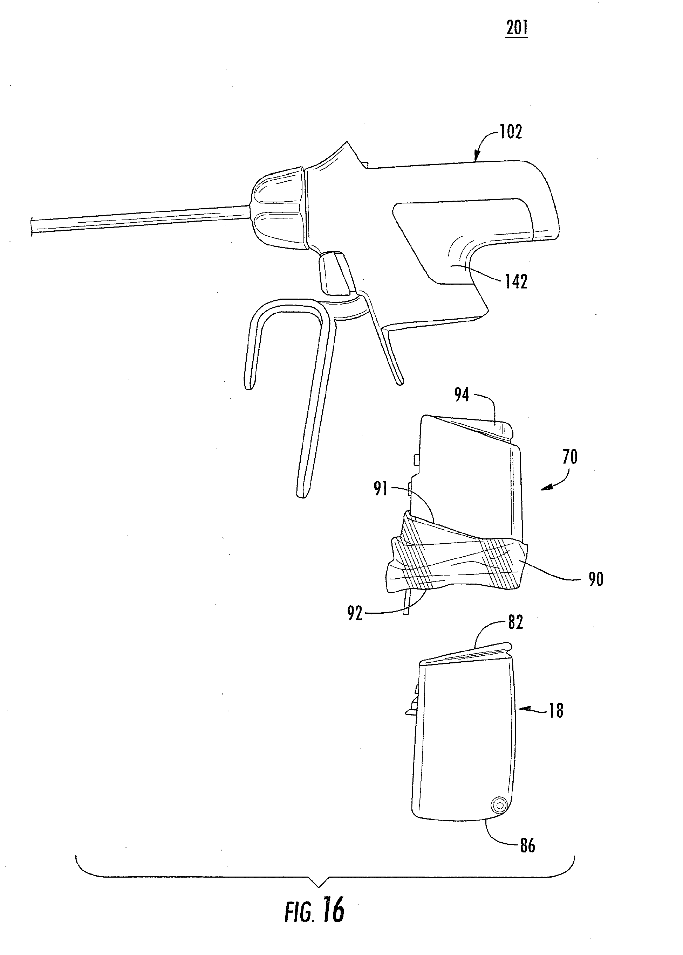

Referring to FIGS. 16-18, another embodiment of a surgical system 201 provided in accordance with the present disclosure incorporates surgical instrument 102 (or any other suitable surgical instrument), battery assembly 18 (or any other suitable energy source), an adapter 70, and aseptic bag 90 (or any other suitable aseptic bag such as those provided hereinabove). Instrument 102, battery assembly 18, and aseptic bag 90 of surgical system 3 are substantially similar to instrument 102, battery assembly 18, and aseptic bag 90 of surgical systems I and 101 described above and, as such, the discussion of surgical system 201 below will be limited to the differences.

Adapter 70 is configured to releasably couple battery assembly 18 to fixed handle 142. It is contemplated that adapter 70 can be configured to allow instrument 2 to interface with battery assemblies of different sizes and shapes and with different configurations of electrical contacts. When provided, adapter 70 is coupled to either fixed handle 142 or battery assembly 18 such that third electrical contacts 94 engage either the first electrical contacts (not shown) of handle 142 or second electrical contacts 82, respectively to electrically couple the contacts of handle 142 with contacts 82 of battery assembly 18.

A first open end 91 of aseptic bag 90 is affixed to adapter 70. First open end 91 can be affixed to adapter 70 using an adhesive connection and/or a snap-fit connection in a manner similar to first open end 91 attaching to fixed handle 42 detailed above. In embodiments, first open end 91 is attached to adaptor 70 by a band (not shown) seating in a groove (not shown) on adaptor 70, similar to band 147 of aseptic bag 190 seating in groove 148 of fixed handle 42 discussed above (see FIG. 10).

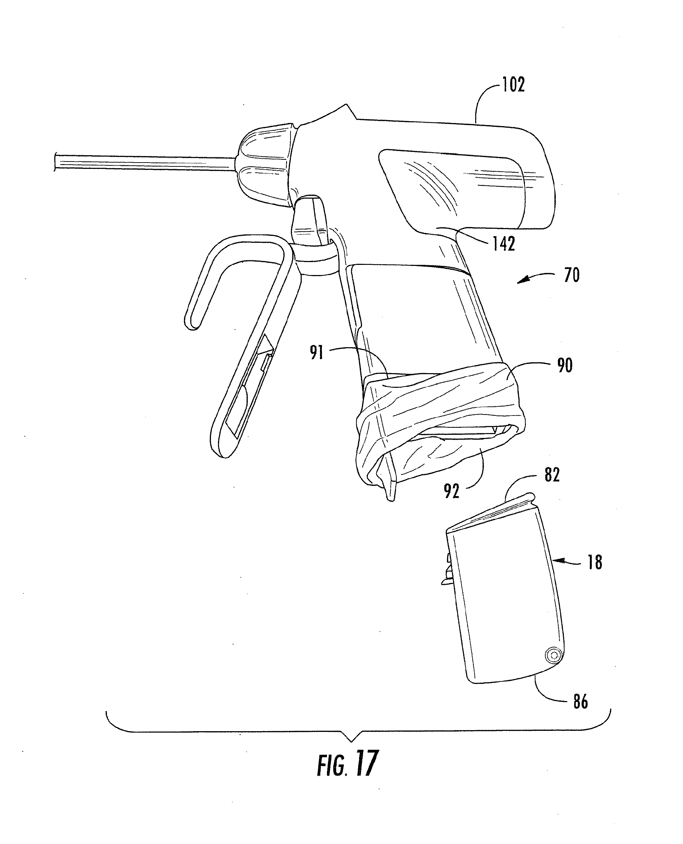

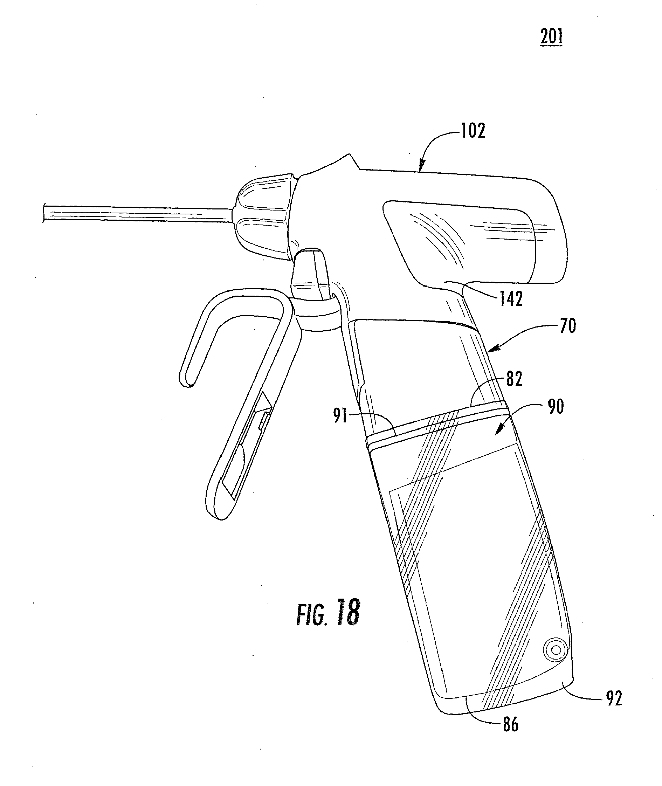

Referring to FIGS. 17 and 18, adaptor 70 is attached to handle 142 of instrument 102 with aseptic bag 90 folded in a low-profile configuration on adaptor 70. Battery assembly 18 is then coupled to adaptor 70 and second open end 92 is extended over battery assembly 18 such that battery assembly 18 is positioned within inner chamber 93 of aseptic bag 90 as shown in FIG. 18.

Referring generally to FIGS. 1-18, according to aspects of the present disclosure, when the battery assembly 18, 118 is coupled to the fixed handle 42, 142, the battery assembly 18, 118 forms a portion of the fixed handle 42, 142. An outer surface of the aseptic bag 90, 190 can also be textured to enhance the gripping surface of the handle assembly 6, 106.

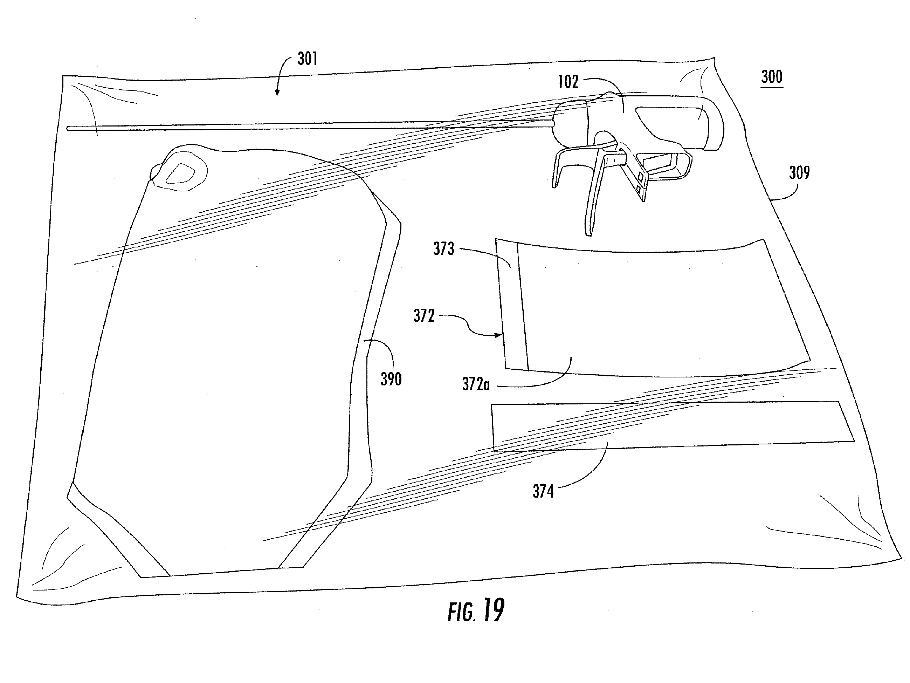

Referring to FIG. 19, an embodiment of a kit 300 provided in accordance with the present disclosure incorporates a surgical system 301 sealed within a membrane 309. Surgical system 301 includes an instrument 102, e.g., an electrosurgical, ultrasonic, or other suitable surgical instrument, a handle wrap 372, and an aseptic bag 390. Surgical system 301 may also include a closure member 374. Examples of instrument 102 and the components thereof are described in detail above.

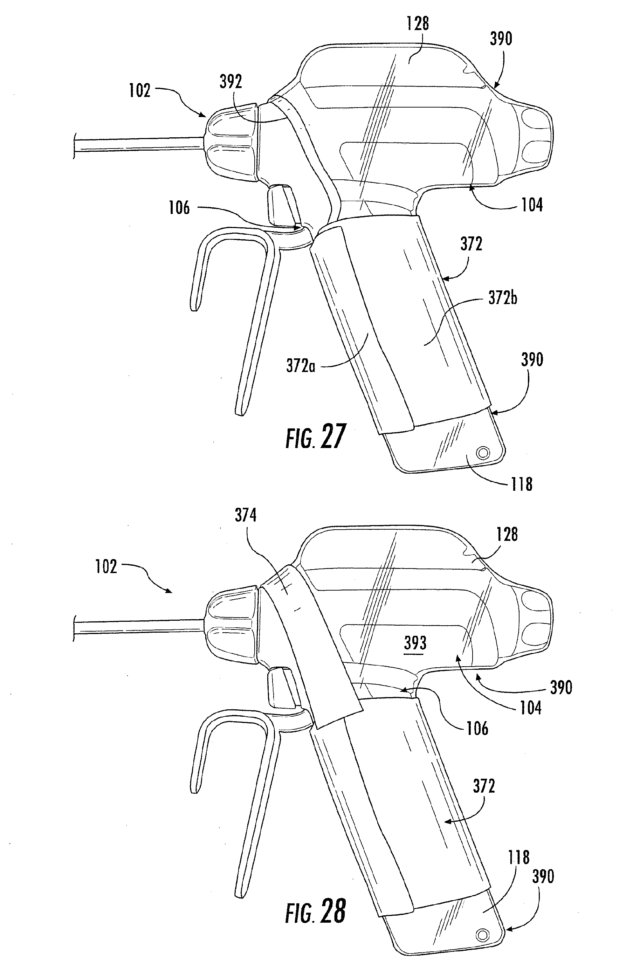

Handle wrap 372 is sized and configured to wrap around a battery assembly 118 (FIG. 3), described in detail below. Handle wrap 372 may include an adhesive strip 373 including a release layer (not shown). In embodiments, an inner surface 372a and/or an outer surface 372b (FIG. 27) of handle wrap 372 is textured to enhance the gripping properties thereof. Handle wrap 372 may be constructed of a low-density foam material; however, other materials are also contemplated. In some embodiments, handle wrap 372 is an adhesive strip, e.g., a strip of surgical tape, configured to be wrapped around battery assembly 118 (FIG. 3) and/or handle assembly 106 one or more times. Handle wrap 372 may be included in kit 300 or be available as a consumable adhesive strip in an operating environment.

Closure member 374 is an adhesive strip configured to releasably secure aseptic bag 390 to instrument 102 as described in detail below. Closure member 374 may be included in kit 300 or be available as a consumable adhesive strip in an operating environment.

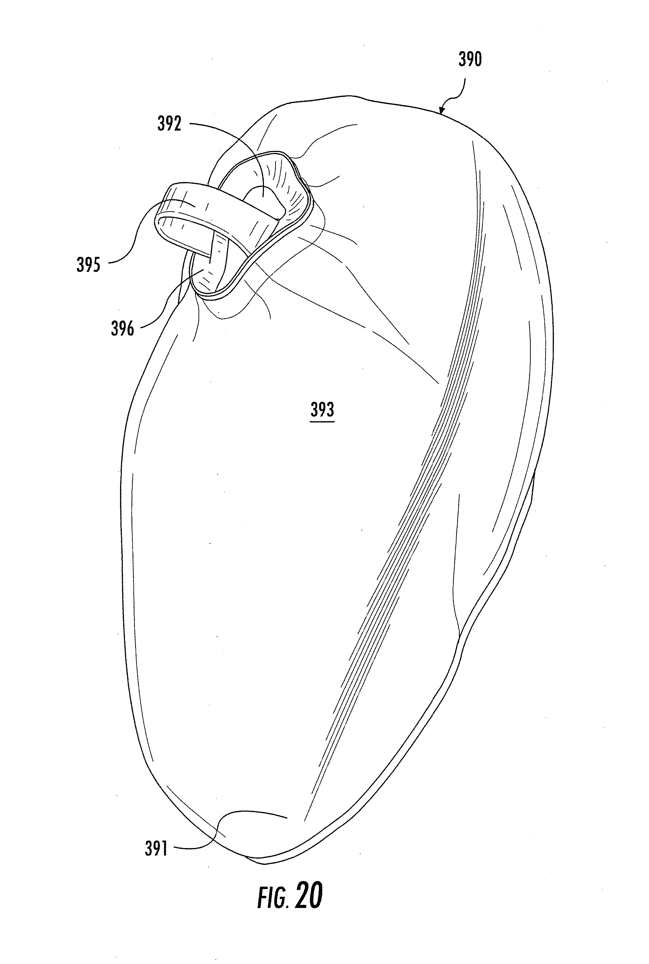

Referring to FIG. 20, aseptic bag 390 includes a closed end 391, an open end or opening 392, and an inner chamber 393 defined therebetween. Opening 392 may include a sealing member 396 that surrounds opening 392 and is configured to bias opening 392 towards a closed position. Aseptic bag 390 may include a strap 395 across opening 392. Strap 395 may be elastically stretchable as described in detail below.

Referring to FIGS. 19-28, the preparation of surgical system 301 for a surgical procedure is detailed below in accordance with the present disclosure. The components of surgical system 301 are removed from kit 300 including instrument 102, handle wrap 372, and aseptic bag 390. To prepare surgical system 301 for a surgical procedure, a sealing member 374, a battery assembly 118 (FIG. 2), and a generator 128 (FIG. 2) may also be required; each of which may be available in an operating environment or be provided as part of kit 300.

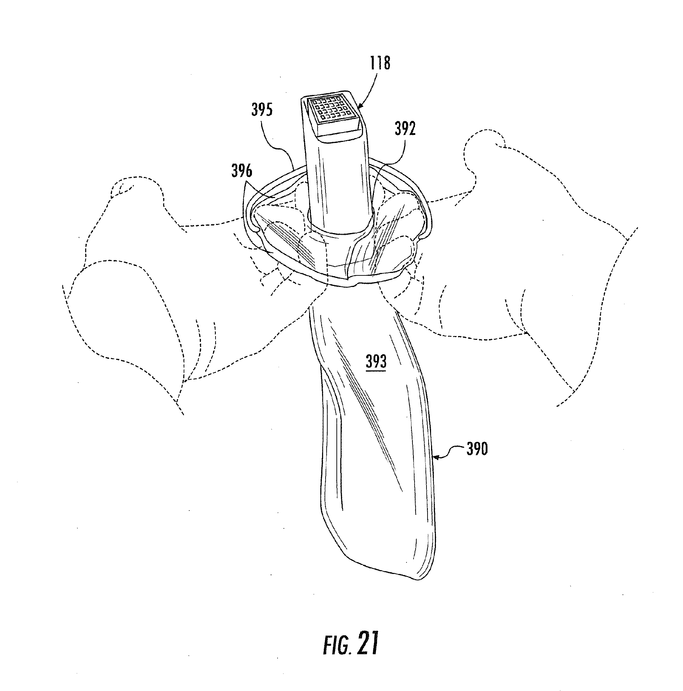

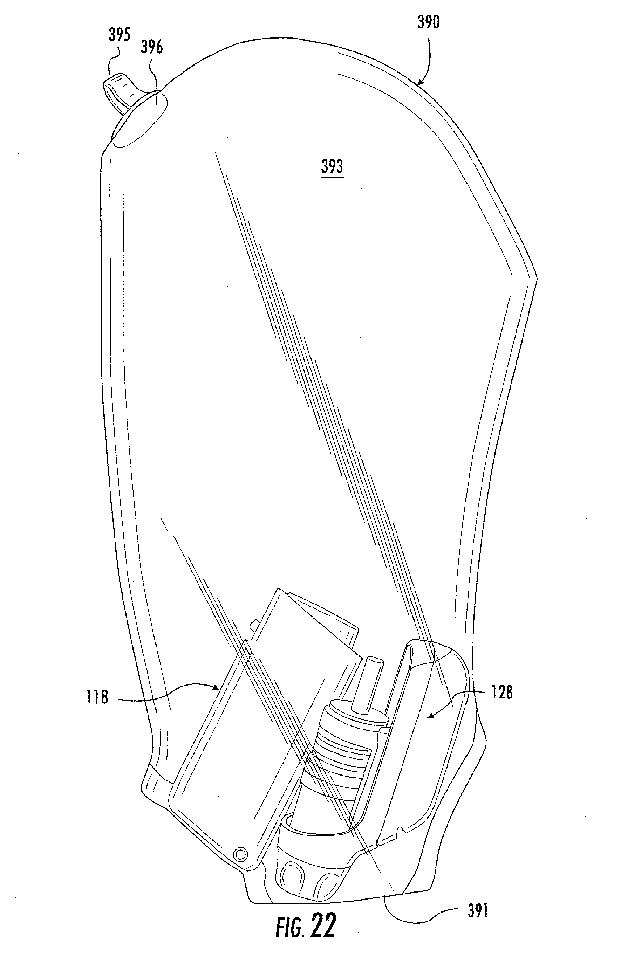

Referring to FIG. 21, opening 392 of aseptic bag 390 is held open against sealing member 396 permitting battery assembly 118 to be inserted through opening 392 and into inner chamber 393 of aseptic bag 390. It will be appreciated that in embodiments where aseptic bag 390 includes strap 395, strap 395 will also be held to one side of opening 392 to permit battery assembly 118 to be inserted through opening 392. Generator 128 (FIG. 2) is also inserted through opening 392 in a manner similar to battery assembly 118 such that battery assembly 118 and generator 128 are both positioned within inner chamber 393 as shown in FIG. 22.

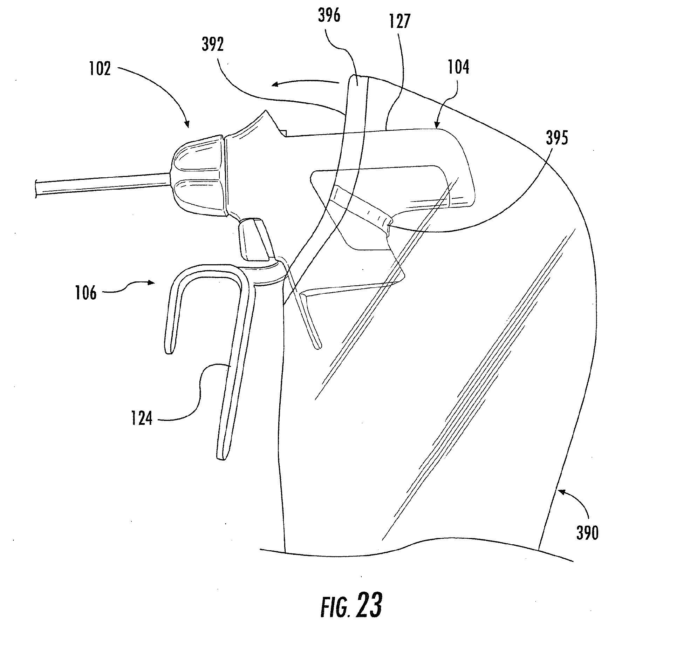

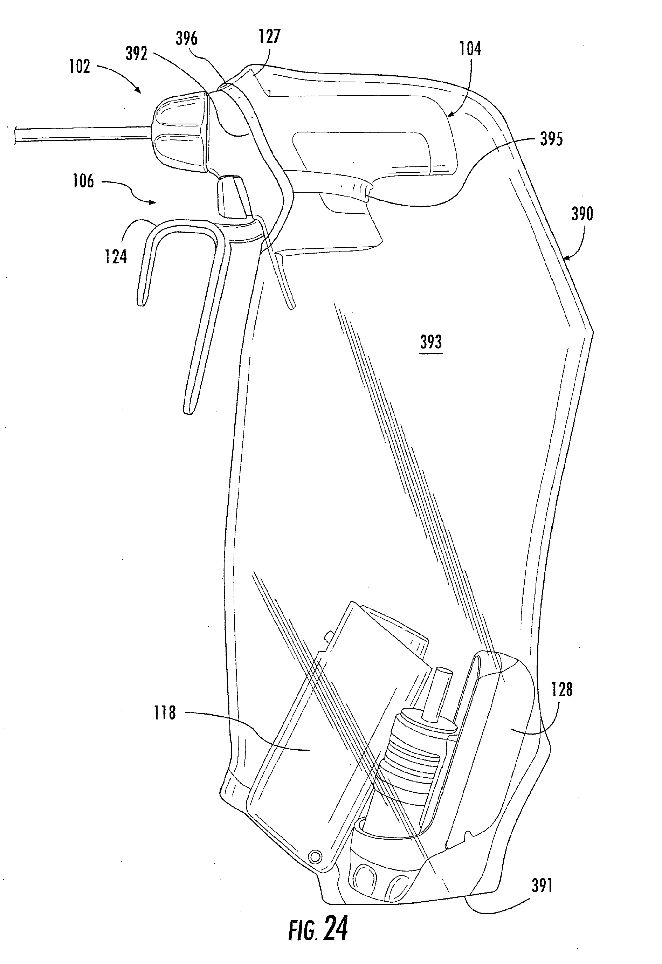

With reference to FIG. 23, the proximal end of housing 104 of instrument 102 is positioned or inserted through opening 392. In embodiments with strap 395, strap 395 is positioned on the proximal end of handle assembly 106 to proximally bias opening 392. The lower portion of opening 392 is positioned adjacent movable handle 124 and the upper portion of opening 392 is positioned distal to a generator slot 127 of housing 104 as shown in FIG. 24. In embodiments with strap 395, the connection points of strap 395 adjacent opening 392 are positioned between the upper and lower portions of opening 392 to bias opening 392 proximally.

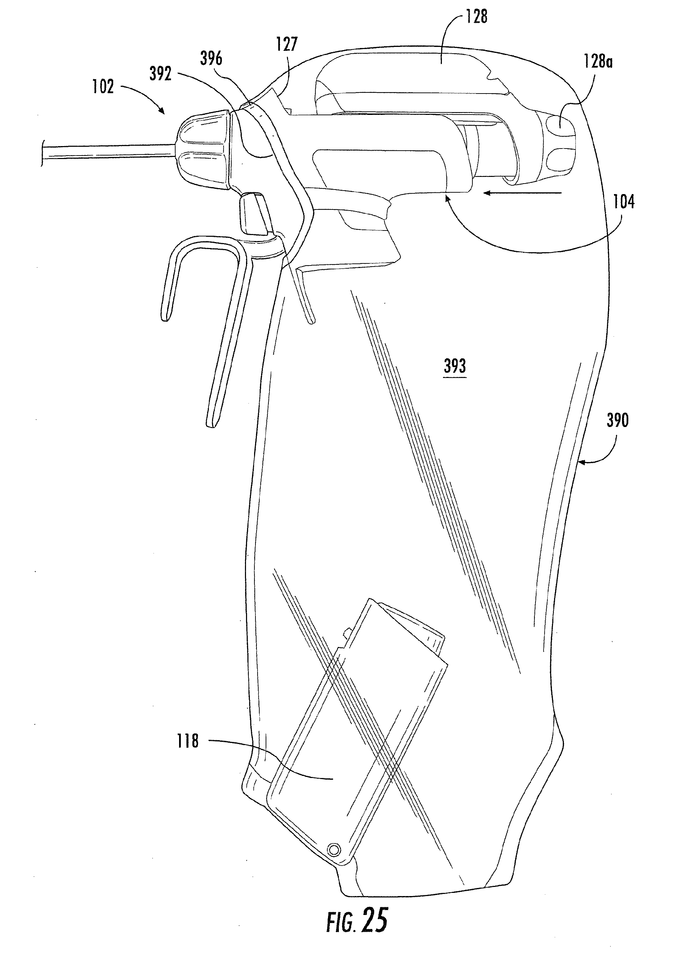

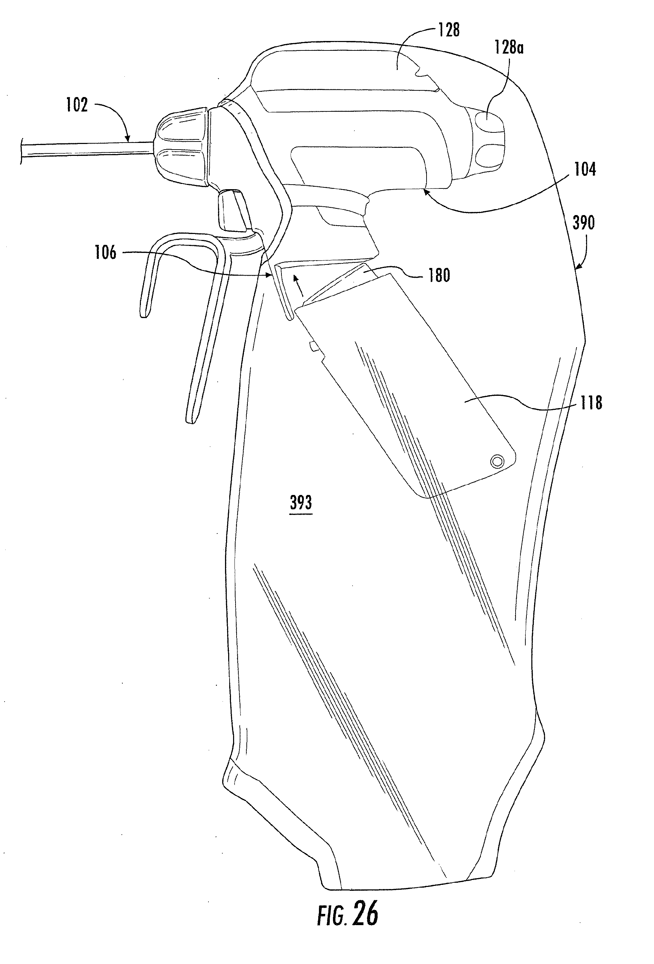

Referring to FIG. 25, generator 128 is manipulated through aseptic bag 390 and slidably inserted into generator slot 127 until the distal end of generator 128 is engaged with housing 104 of instrument 102. A rotatable knob 128a may be positioned on the proximal end of generator 128. Rotatable knob 128a may be rotated to secure generator 128 to housing 104 as shown in FIG. 26. Rotational securement is suitable for use with respect to ultrasonic surgical instruments wherein the generator further includes a transducer, although such a configuration may also be utilized with other surgical instruments. Rotatable knob 128a may be operatively associated with threads (not shown) to engage corresponding threads (not shown) of housing 104. Other securement mechanisms are also contemplated to secure generator 128 to housing 104, e.g., snap fit, etc.

With continued reference to FIG. 26, battery assembly 118 is manipulated through aseptic bag 390 to attach battery assembly 118 to handle assembly 106. Contact cap 180 engages handle assembly 106 to engage second electrical contacts 182 (FIG. 3) with first electrical contacts (not shown) of instrument 102 to deliver energy from battery assembly 118 to instrument 102. Alternatively or additionally, battery assembly 118 may be secured to instrument 102 prior to securement of generator 128 thereto.

Referring to FIG. 27, handle wrap 372 is wrapped around battery assembly 118 and handle assembly 104 about the exterior of aseptic bag 390 such that outer surface 372b (FIG. 27) of handle wrap 372 is exposed or outwardly-facing. Adhesive surface 372a of handle wrap 372 may be used to secure handle wrap 372 to itself and/or to aseptic bag 390. Excess material of aseptic bag 390 may be positioned between battery assembly 118 and handle wrap 372 such that aseptic bag 390 substantially conforms to shape of surgical instrument 102, generator 128, and battery assembly 118.

Referring now to FIG. 28, closure member 374 is placed over the interface of opening 392 (FIG. 27) of aseptic bag 390 and housing 104 of instrument 102 in a sealing relationship with both aseptic bag 390 and instrument 102 to encapsulate battery assembly 118 and generator 128 within inner chamber 393. Alternatively or additionally, sealing member 396 (FIG. 20) of aseptic bag 390 may be itself sufficient to seal battery assembly 118 and generator 128 within inner chamber 393. When battery assembly 118 and generator 128 are sealed within inner chamber 393 instrument 102 is prepared for a surgical procedure.

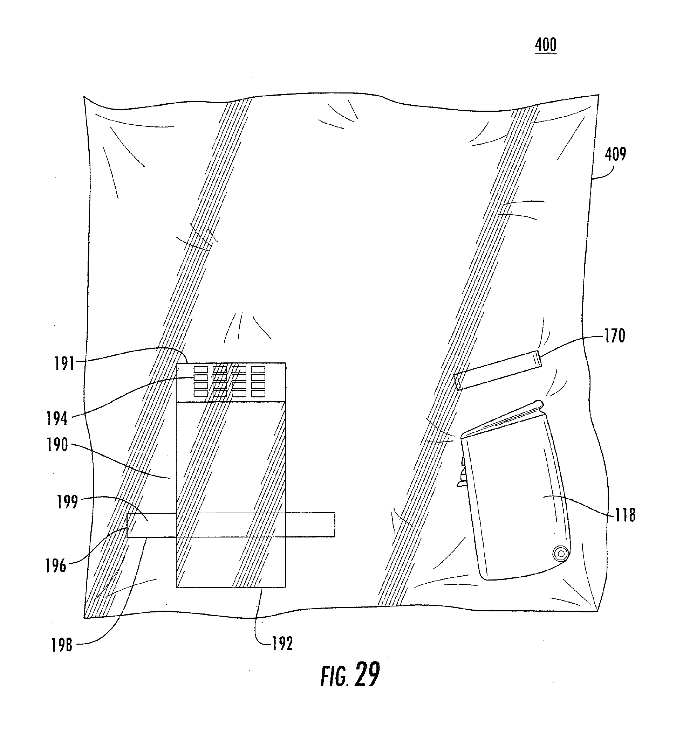

With reference to FIG. 29, another embodiment of a kit 400 provided in accordance with the present disclosure incorporates an aseptic bag 190 and battery assembly 118 sealed within a membrane 409. The aseptic bag 190 and battery assembly 118 may be in a single chamber of membrane 409 or in separate chambers defined within membrane 409. It will be appreciated that membrane 409 encapsulates aseptic bag 190 and battery assembly 118 such that each may be sterilized before being encapsulated within membrane 409 and transported to a medical facility or operating theater in a sterile manner. Aseptic bag 190 includes a closed end 191, an open end 192, third electrical contacts 194 near a closed end 191, and a sealing member 196. Sealing member 196 includes an adhesive layer (not shown) covered by a releasable cover member 199. Aseptic bag 190 is sized and configured to encapsulate battery assembly 118 therewith. Kit 400 may further include any additional components such as those detailed above. Other similar kits may be provided from any of the other embodiments detailed above or combinations thereof.

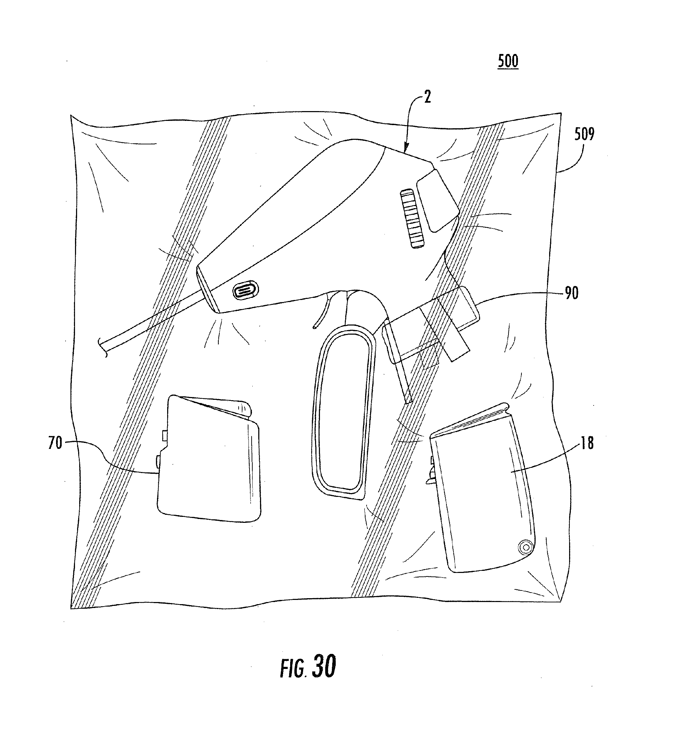

With reference to FIG. 30, another embodiment of a kit 500 provided in accordance with the present disclosure incorporates instrument 2 (although other instruments are also contemplated), battery assembly 18, and aseptic bag 90 affixed to the instrument 2 sealed within a membrane 509. In embodiments, kit 500 further includes an adapter 70. Kit 500 may further include any additional components such as those detailed above. Moreover, membrane 509 of kit 500 is substantially similar to membrane 409 of kit 400 and will not be discussed in further detail. Other similar kits may be provided from any of the embodiments detailed above or combinations thereof.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the claimed invention. Therefore, the above description should not be construed as limiting, but merely as exemplifications of particular embodiments. Those skilled in the art will envision other modifications within the scope and spirit of the claims appended hereto.

## Claims

1. A method of preparing a surgical system for surgery, comprising:
inserting a battery assembly into an inner chamber of an aseptic bag through an open end of the aseptic bag;
positioning a portion of a housing of a surgical instrument through the open end of the aseptic bag;
coupling the battery assembly to the surgical instrument while the battery assembly is within the inner chamber; and
sealing the open end of the aseptic bag to the housing of the surgical instrument to encapsulate the battery assembly within the inner chamber of the aseptic bag.

2. The method of claim 0 further including inserting a generator into the inner chamber of the aseptic bag through the open end of the aseptic bag before sealing the open end of the aseptic bag, and sealing the open end of the aseptic bag further includes encapsulating the generator within the inner chamber of the aseptic bag.

3. The method of claim 0 further including securing the generator in a slot defined by the housing of the surgical instrument after positioning a portion of a housing of a surgical instrument through the open end of the aseptic bag.

4. The method of claim 1, 2 or 3, further including affixing a closure member around the open end of the aseptic bag to seal the open end of the aseptic bag to the surgical instrument.

5. The method of any preceding claim further including wrapping the battery assembly and a portion of the aseptic bag with a handle wrapper after sealing the open end of the aseptic bag to the housing of the surgical instrument, optionally
wherein the handle wrapper includes an adhesive surface, and wherein wrapping the battery assembly includes affixing the adhesive surface of the handle wrapper to another portion of the handle wrapper to secure the handle wrapper around the battery assembly and a portion of the aseptic bag, and further optionally
further including gathering loose portions of the aseptic bag around the battery assembly after coupling the battery assembly to the surgical instrument and before wrapping the battery assembly and the portion of the aseptic bag with the handle wrapper.

6. The method of any preceding claim, wherein the open end of the aseptic bag includes a strap, and wherein positioning the portion of the housing of the surgical instrument through the open end of the aseptic bag includes positioning the strap over a proximal portion of a handle assembly of the surgical instrument to bias the open end proximally.

7. The method of any preceding claim further including removing the surgical instrument and the aseptic bag from a surgical kit before inserting the battery assembly into the inner chamber of the aseptic bag.

8. A method of preparing for surgery, comprising:
coupling an energy source to a surgical instrument;
extending an aseptic bag over the energy source; and
sealing an open end of the aseptic bag to encapsulate the energy source within an inner chamber of the aseptic bag.

9. The method of claim 8, wherein coupling includes electrically coupling electrical contacts of the aseptic bag with electrical contacts of the energy source.

10. The method of claim 8, wherein extending includes pulling a tab disposed on the aseptic bag to extend the aseptic bag over the energy source.

11. The method of claim 10, wherein sealing includes removing a releasable cover member from the tab to expose an adhesive surface and adhering the tab to the aseptic bag.

12. The method of claim 8, 10 or 11, wherein sealing includes engaging the open end of the aseptic bag with a fixed handle of the surgical instrument.

13. The method of any one of claims 8 to 12, wherein the surgical instrument is energizable to deliver energy from the energy source to tissue.

14. A surgical system including;
a surgical instrument,
a battery assembly,
an aseptic bag, and
a sealing member, wherein :
the battery assembly is configured to deliver energy to the surgical instrument and is removably engagable with the surgical instrument, the aseptic bag includes an open end and an inner chamber, the battery assembly is positionable within the inner chamber of the aseptic bag, the sealing member is in contact with the aseptic bag and the housing of the surgical instrument to seal the open end of the aseptic bag to encapsulate the battery assembly within the inner chamber while still permitting energy delivery from the battery assembly to the surgical instrument.

15. A surgical system including:
a surgical instrument,
an energy source,
an aseptic bag, and
a sealing member, wherein:
the surgical instrument includes first electrical contacts, the energy source includes second electrical contacts configured to electrically couple to the first electrical contacts of the surgical instrument, the energy source is deliverable to energy to the surgical instrument and is removably engagable with the surgical instrument, the surgical instrument is able to deliver the energy to tissue and/or use the energy to power the surgical instrument, the aseptic bag includes an open end and an inner chamber, the energy source is positionable within the inner chamber of the aseptic bag, the sealing member is affixed to the aseptic bag and configured to seal the open end of the aseptic bag to encapsulate the energy source within the inner chamber while still permitting energy delivery via the first and second electrical contacts.

16. A surgical system including:
a surgical instrument,
an energy source,
an aseptic bag, and
a sealing member, wherein:
the surgical instrument defines an attachment portion and includes first electrical contacts adjacent to the attachment portion, the surgical instrument is able to deliver the energy to tissue and/or use the energy to power the surgical instrument, the energy source removably engages the attachment portion of the surgical instrument, the energy source includes second electrical contacts configured to electrically couple to the first electrical contacts of the energy source when the energy source engages the attachment portion of the surgical instrument, the energy source is configured to deliver energy to the surgical device through the first and second electrical contacts, the aseptic bag includes first and second open ends and an inner chamber between the first and second ends, the first open end is coupled to the surgical instrument, the first open end is able to be coupled to the surgical instrument near the electrical contacts of the surgical instrument such that the electrical contacts are within the inner chamber, the energy source is positionable within the inner chamber, the sealing member is affixed to the aseptic bag and is configured to seal the second open end of the aseptic bag to encapsulate the energy source within the inner chamber.
